Europäisches Patentamt

European Patent Office

Office européen des brevets

(11) Publication number: **0 472 237 A1**

# EUROPEAN PATENT APPLICATION

(21) Application number: **91202075.7**

(22) Date of filing: **14.08.91**

(51) Int. Cl.5: **A61L 27/00**, C08L 23/06,
//(C08L23/06,C08K3:32)

(30) Priority: **21.08.90 NL 9001847**

(43) Date of publication of application:
**26.02.92 Bulletin 92/09**

(84) Designated Contracting States:
**AT BE CH DE DK ES FR GB GR IT LI LU NL SE**

(71) Applicant: **DSM N.V.**
**Het Overloon 1**
**NL-6411 TE Heerlen(NL)**

(72) Inventor: **Calis, Gijsbertus Hendrikus Maria**
**Burg. Kengenstraat 60**
**NL-6336 BK Nuth(NL)**
Inventor: **Van Unen, Lambert Henri Theodoor**
**Spechtstraat 12**
**NL-6414 XH Heerlen(NL)**

(74) Representative: **Hoogstraten, Willem Cornelis**
**Roeland et al**
**OCTROOIBUREAU DSM Postbus 9**
**NL-6160 MA Geleen(NL)**

(54) Prosthesis from polyethylene filled with an inorganic filler.

(57) Prosthesis formed from a composition comprising ultra-high molecular weight polyethylene with a low entanglement density and an inorganic filler and a process for producing such a prosthesis by the moulding of an ultra-high molecular weight polyethylene with a low entanglement density, filled with an inorganic filler, at a temperature of between $T_m - 25\,°C$ and $T_m + 60\,°C$, where Tm is the melting point of the filled ultra-high molecular weight polyethylene.

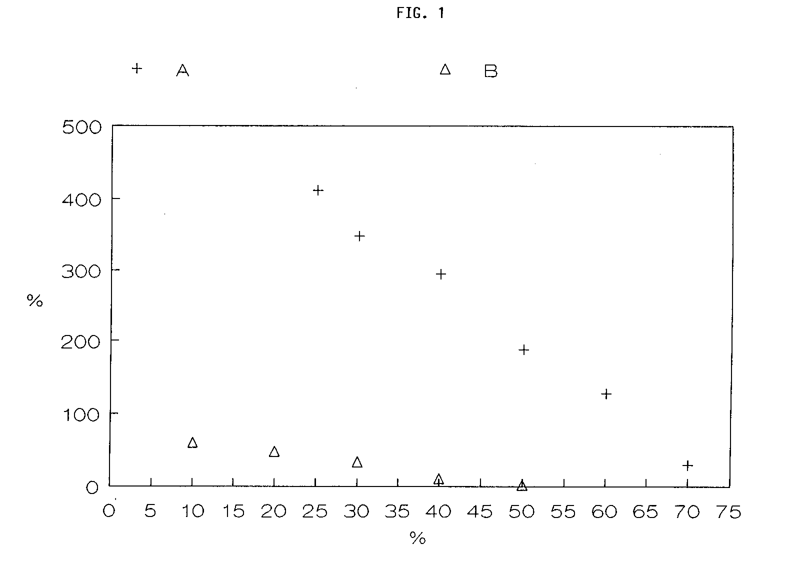

FIG. 1

The invention relates to a prosthesis from poly-ethylene filled with an inorganic filler.

Such a prosthesis is known from the European patent application EP-A-49720, in which polyethylene with a weight-average molecular weight of about 300,000 kg/kmole is mixed in its melted state with the inorganic filler. Then, after cooling, a raw material is obtained in the form in which the melt has solidified or optionally, after milling the solidified melt, as a granulate. From this raw material an end product, for instance a bone prosthesis, is then produced by melt moulding.

The disadvantage of this known prosthesis is that, in consequence of the repeated melting, necessary for obtaining a homogeneous distribution of the filler, the weight-average molecular weight of the polyethylene in the prosthesis turns out to be much lower than that of the starting material. As a consequence, the favourable properties of the starting material that go with a high molecular weight, such as the high wear resistance and toughness, are only partly found back in the end product. This is particularly the case with high filler loads, because in that case the filler must be added phase by phase, in each phase of which the polyethylene must be cooled and melted again. The loss of molecular weight under the influence of the production conditions may increase to more than 50%, as known also from the magazine entitled Plastics and Rubber Processing and Applications, Vol. 4, no. 3, 1984, pp. 261-269. Despite the wide limits for the molecular weight of the polyethylene to be applied as defined in the European patent application EP-A-49720, it has been found that the known prosthesis can be produced only from polyethylene with a weight-average molecular weight of 400,000 kg/kmole at most as starting material, because, with higher molecular weights, the very high melt viscosity makes it virtually impossible for the inorganic material to be homogeneously admixed. Neither are at this moment any other processes known for the production of such a prosthesis from polyethylene with a weight-average molecular weight of above about 500,000 kg/kmole, while the properties of this material make it highly suited for use in bone prostheses and consequently the availability of prostheses produced with this material is highly desirable.

The object of the invention is to provide a prosthesis that does not have the said disadvantages and limitations, or only to a considerably lower degree.

This object is achieved according to the invention in that the prosthesis is formed from a composition comprising ultra- high molecular weight polyethylene with a low entanglement density and the inorganic filler.

The prosthesis according to the invention has the favourable properties of ultra-high molecular weight polyethylene, hereinafter referred to as UHMWPE, of which the most conspicuous in this use are biocompatibility, a high toughness, impact resistance and wear resistance and a low coefficient of friction. These properties are a particular feature of polyethylene with a weight-average molecular weight of at least 500,000 kg/kmole and more specifically of at least 1,000,000 kg/kmole. Moreover, the filler load of the polyethylene in the prosthesis may vary within wide limits to allow an optimum adjustment to the mechanical properties of the part of the human or animal body that is replaced by the prosthesis or which the prosthesis is to be affixed to. A further advantage of the prosthesis according to the invention is that the elongation at break, which is a measure of the brittleness of the material of the prosthesis, has considerably higher values than that of the known prosthesis with the same filler load, which allows much higher filler loads to be used without an unacceptable brittleness of the prosthesis arising. This widening of the range of usable filler loads also increases the possibilities of prostheses adjusted to suit the mechanical requirements imposed by a concrete application.

The prosthesis according to the invention is meant to be an artificial replacement of parts of the human or animal body, both internally and externally, particularly of bone, bone parts, joints, parts of joints, provisions for affixing natural or artificial tendons to the said parts of the body and denture elements or parts thereof. The prosthesis consists of UHMWPE with a low entanglement density, filled with an inorganic filler, hereinafter referred to as filled polyethylene. The inorganic substances that are used in the prosthesis must be accepted by the organism without giving rise to, for instance, toxic, inflammatory or rejection reactions. The amount of inorganic material contained in the polyethylene influences to a considerable degree the modulus of elasticity thereof. The fact is that the presence of the organic material up to an amount of 80% (vol.) calculated on the total amount of inorganic material and polyethylene is known to produce filled polyethylene with a modulus of elasticity corresponding with that of the various parts of the human and/or animal body that may qualify for being replaced by prostheses or for being affixed thereto. As the prosthesis is meant to replace bones or related parts, it is an advantage to choose a filler with a high degree of similarity to the bone material. So by making a suitable choice of the inorganic filler it is possible both to influence the stiffness of the prosthesis and to improve the knitting of the prosthesis to the existing tissue. That is why preference is given to substantially calcium salts as inorganic material, and the calcium salt most preferably comprises a calcium phosphate, hydroxyapatite or fluorapatite. In addition to synthetic fillers, fillers of a natural origin can be used also such as, for instance, the mineral components of natural bone material. These consist to a high degree of hydroxyapatite and can be obtained, for instance, by the ashing of bone material. In order to promote the knitting of the prosthesis to the surrounding bone or bone-like tissue it may be an advantage to apply an extra amount of filler on the surface of the prosthesis or to apply, on the surface, filled polyethylene with a relatively high filler load.

The prosthesis is moulded from UHMWPE. By UHMWPE is meant herein a linear polyethylene with fewer than 1 side chain per 100 carbon atoms and preferably with fewer than 1 side chain per 300 carbon atoms and such a polyethylene as is capable also of containing minor amounts, preferably less than 5% (mole), of one or more other alkenes, such as propylene, butylene, pentene, hexene, 4-methyl-pentene, octene, etc., polymerized therewith, which polyethylene or copolymer of ethylene has a weight-average molecular weight of at least 500,000 kg/kmole. The polyethylene may further contain minor amounts, preferably 25% (wt) at most, of one or more other polymers, particularly an alkene-1 polymer, such as polypropylene, polybutadiene or a copolymer of propylene with a minor amount of ethylene. Such an UHMWPE can be produced, for instance, by means of a Ziegler or a Phillips process while applying suitable catalysts and under polymerization conditions known in the art.

UHMWPE is highly compatible with the human and animal organisms and is not affected, dissolved or otherwise weakened by the effects of substances present in the human or animal organisms, which substances it may come into contact with after implantation. Although these favourable properties are already important features of UHMWPE with a weight-average molecular weight of at least 500,000 kg/kmole, preference is given to the use of UHMWPE having a weight-average molecular weight of at least 1,000,000 kg/kmole, and most preference to the use of UHMWPE having a weight-average molecular weight of at least 1,500,000 kg/kmole, because the properties of these in particular guarantee the necessary long lives of the prostheses produced from this material.

The prosthesis is moulded from UHMWPE with a low entanglement density. The entanglement density concerns the degree to which the polyethylene molecules, which are very long with the high molecular weights applied, are entangled with each other.

For the direct determination of the degree of entanglement no method is known, but there is a relationship between the maximum draw ratio of the UHMWPE below its melting point and the degree of entanglement. Low-entanglement material is characterized by a molecular drawability below its melting point of at least 10x and preferably at least 20x, the tensile strength and the modulus of elasticity showing a substantial increase during the drawing of an article consisting of the low-entanglement polyethylene. Polyethylene subjected to temperatures above its melting point does not have this property. This low etanglement density has been found to be accompanied by a special and advantageous property of the polyethylene, viz. a very rapid and strong adhesion between particles or layers of the polyethylene even at low temperatures. Under static circumstances, such as for instance compression, this strong adhesion is achieved already at temperatures just a little, for instance about 15°C, above the melting point of the polyethylene, though even at lower temperatures, for instance from about 25°C below this melting point, continuous and well-manageable moulded articles can be obtained. By applying processes involving a strong deformation of the polyethylene, for instance under the influence of shear forces, as is the case in, for instance, extrusion or injection moulding, it has been found that fully continuous articles from low-entanglement polyethylene particles can be moulded already at temperatures below the melting point of the polyethylene, for instance from 25°C below this melting point. Low-entanglement polyethylene filled with inorganic material has been found to possess this favourable property also. Therefore, during the production process, a prosthesis made from this filled low-entanglement polyethylene will only have to be exposed to relatively low temperatures, and that for a short period of time, so that thermal degradation of the polyethylene and the resulting molecular weight reduction of the UHMWPE and deterioration of the favourable properties are avoided.

The morphology of a polyethylene article obtained by the compression of low-entanglement UHMWPE at a temperature above its melting point has been found to differ markedly from that of a polyethylene article obtained by compressing UHMWPE not having a low density of entanglement. The fact is that transmitted light microscopy on articles moulded from UHMWPE powder has revealed that in the former case the polyethylene has a homogeneous structure, whereas in the latter case the boundaries of the powder particles are clearly visible. A comparable difference in morphology is visible on the surface of the material after the material has been made to swell in a solvent.

Prostheses must have mechanical properties in agreement with the function of that part of the body which they replace as well as those parts of the body which they are affixed or anchored to.

The most characteristic property in this connection is the stiffness of the prosthesis, expressed in the value of the modulus of elasticity. The desired value of the modulus of elasticity that guarantees correct agreement has been found in many cases to differ from one place in the prosthesis to another.

For instance, in EP-A-19.044 the insight exists that the modulus of elasticity of the stem section, that is the section that is anchored in the femur, should decrease from the distal to the proximal end. However, in said application the use of low-entanglement UHMWPE filled with inorganic material as raw material for such a prosthesis is neither disclosed nor suggested. Moreover, in a hip prosthesis, the desired value of the modulus of elasticity of the stem section is found in the longitudinal direction thereof not to show a regular decrease, but to vary according to a complex pattern. The prosthesis according to the invention now makes it possible to provide such a fluctuating modulus of elasticity in that the prosthesis is built up from parts with different filler loads. The existence of a relationship between the modulus of elasticity of filled polyethylene and the amount of filler used is known per se, for instance from the article by W. Bonfield et al. in 'Biomaterials and Biomechanics', pages 421-426, edited by P. Ducheyne et al., Elsevier Science Publishers, Amsterdam, 1984. The size of the filler particles, too, and their shapes, for instance the ratio between their length and their thickness and width, have been found to influence the modulus of elasticity of filled UHMWPE.

For instance, at a specific filler load, the highest modulus of elasticity is obtained with the smallest filler particles. Using the above-mentioned relationships, it is possible for the modulus of elasticity of filled polyethylene to be set accurately and reproducibly within wide limits.

Inorganic material particles of all shapes can be used. Suitable are granular particles, round as well as angular, the diameter of which is preferably 100 $\mu$ m at most, and elongated and plate-shaped particles, of which the dimensions in one of the directions is not more than 20 $\mu$ m and in the two other directions not more than 500 $\mu$ m. Short fibres, too, can be used, though in that case it is not possible in the production of the prosthesis or of parts thereof to proceed from fine filled powder, but it is advantageous to use UHMWPE filled with inorganic fibres in the form of filled film-shaped layers.

The variation of the modulus in a prosthesis by using a filled polymer with different filler loads is known per se from DE-OS-3005265, in which this technique is used to achieve a step-by-step transition of the relatively high modulus of the core of the prosthesis used in it and the relatively low modulus of the bone cement with which the prosthesis is affixed to the bone. To solve this problem, the core is wrapped in sheets or plates of a polymer, particularly polymethylmethacrylate, with different moduli. However, that patent publication lacks the insight to use filled polyethylene with a low degree of entanglement and to produce the whole prosthesis from similar material with already the correct modulus. Moreover, said patent publication does not provide a solution for the real problem, viz. the adjustment of the prosthesis to the variations of the modulus of elasticity in the longitudinal direction of the bone which the prosthesis is inserted in.

The invention also relates to a process for the production of a prosthesis as described hereinbefore by moulding an UHMWPE with a low entanglement density, filled with an inorganic filler, at a temperature of between $T_m$-25$^\circ$C and $T_m$+60$^\circ$C, where $T_m$ is the melting point of the filled UHMWPE.

The moulding of the UHMWPE with a low entanglement density, filled with an inorganic filler, can be effected according to processes known per se. Examples of these are the filling of a mould with powder, granulate or film-shaped layers consisting of the material and the compression thereof at elevated temperature in order thus to obtain a continuous article of the desired shape consisting of the material. The material can be supplied also in the form of a powder or granulate to an extruder, to an injection moulding machine or to other devices known per se for the moulding of plasticized materials, and using these an article can be produced consisting of the material. The article thus produced may be a complete prosthesis, as well as a part of a prosthesis, which is later on brought together with other parts to form a complete prosthesis. This bringing together of the parts of a prosthesis for the formation of a complete prosthesis can be achieved by means of the above-mentioned known techniques by which the parts have been moulded also. A major distinction between the production of a part of a prosthesis and of a complete prosthesis is the temperature at which the production takes place. It has been found that by moulding at a temperature below the melting point of the filled polyethylene, for instance from 25$^\circ$C below this melting point, moulded articles can be obtained with sufficient dimensional stability and continuity which still have the said low entanglement density so important for the invention. In a next step, the various moulded articles, which in this connection can be understood also to include filled UHMWPE powder, can then be compressed under the conditions described above to form a complete continuous prosthesis. The production of a part of a prosthesis is therefore effected preferably at a temperature ranging from 25$^\circ$C below the melting point to the melting point of the filled polyethylene, so that the good adhesion is retained.

It is desirable, however, for the production of a complete prosthesis, either directly as one whole, or from separate parts, to take place at a temperature ranging from the melting point of the filled polyethylene to at most 60$^\circ$C above this point. At this temperature, the low density of entanglement and its related properties are lost, and the final prosthesis again possesses all the favourable properties that make UHMWPE so excellently suited for use in live material. An important difference is that, after the heating of low-entanglement UHMWPE to above its melting point, the UHMWPE has become much tougher. Neither are the good flow properties under mild conditions found to be present any more after heating the UHMWPE to above its melting point and cooling it, and owing to the known poor melt flow properties of UHMWPE without a low entanglement density, the final prosthesis again has a high dimensional stability at elevated temperature. The good flow properties that are so favourable for the production of the prosthesis are undesirable in the final prosthesis in connection with the danger of deformation in the case of a possible exposure to elevated temperatures, for instance during subsequent processing of the prosthesis. In order to avoid thermal degradation, the filled polyethylene must not be heated to beyond 60°C above its melting point. It is always an advantage so to choose the processing temperature and the time during which the filled polyethylene is exposed to this temperature, particularly to a temperature above its melting point, that the material is exposed to the lowest possible heat load.

Polyethylene with a low entanglement density, filled with inorganic material, in a form suited for the process, such as for instance powder, granules or film-shaped layers, can be obtained, for instance, by gelling a dilute solution of UHMWPE in a gelling solvent with the desired amount of inorganic filler dispersed in it and, after the complete or partial removal, if so desired, of the solvent therefrom and while applying the known techniques, such as milling, by reducing the size of the resulting gel to form a powder or granules having the desired filler load and having the desired dimensions. Very fine powders can be obtained if the gel filled with the inorganic material, which gel yet contains a substantial part of the solvent, is reduced in size at a temperature below its solidification point, the gel being very brittle there. It is possible also, in the manner described above, to produce a filled gel film that can be used, after removal of the solvent, as a filled UHMWPE layer in the moulding of the prosthesis in the process according to the invention. The invention also relates to the above-mentioned processes for the production of powder, granules or films of UHMWPE with a low entanglement density, filled with inorganic filler.

Fine filled powders are advantageous for completely filling the moulds used in the production of the prosthesis from the powder and in filling up the remaining spaces in the production of a prosthesis built up from separate parts. That is why the particle size of the filled polyethylene powder preferably ranges between 30 $\mu$ m and 5 $\mu$ m and more preferably between 50 $\mu$ m and 500 $\mu$ m, though larger particles, too, have the favourable properties of the low-entanglement polyethylene. The invention therefore also relates to a powder composition comprising an ultra-high molecular weight polyethylene with a low entanglement density, filled with an inorganic filler, preferably suited for the production of a prosthesis according to the invention.

It is desirable for the modulus of elasticity in every part of the prosthesis produced in this manner to have a value compatible with the characteristics of the tissue which the prosthesis is affixed to and of the load which the prosthesis will, in situ, be exposed to. The prosthesis is therefore preferably composed of UHMWPE with different filler loads. This makes it possible in every part of the prosthesis for particularly the modulus of elasticity to be regulated by the filler load of the UHMWPE used in that part and, in addition, also by the form in which the inorganic material is applied. For the formation of substantially horizontal layers, the mould can successively be charged with the required amounts of filled polyethylene with the desired filler loads in, for instance, the form of a powder or of granules. For the formation of more complex structures, in which process the casting and flow behaviour of powder or granules hampers the application and fixation thereof in a particular form, preference should be given to building up the prosthesis from moulded articles previously compressed, extruded or injection-moulded, at a temperature of between $T_m$-25°C and $T_m$, into a continuous whole from UHMWPE powder with the desired filler load. The prosthesis can be built up also from layers consisting of UHMWPE film filled to the desired filler load with inorganic powder and having the desired thickness, or of pieces cut therefrom to the desired shapes. The prosthesis can also be built up from a combination of filled film layers, moulded articles and powder and, if so desired, filled or non-filled fibres, too. In all said options for building up the prosthesis, a good adhesion is always obtained with the low-entanglement grade of the UHMWPE employed.

The invention is elucidated by means of the following examples without, however, being limited thereto. The parameters mentioned in the examples are determined as described below.

The modulus of elasticity and the elongation at break are determined on the basis of the stress-strain curve measured at room temperature using a Zwick 1435 tensile testing machine on samples having a clamping length of 50 mm at a drawing speed of 50 mm/sec.

Example I

Polyethylene with an Intrinsic Viscosity of 11.9 dl/g, corresponding with a weight-average molecular weight of 1,600,000 kg/kmole, is suspended in xylene in a concentration of 3% (wt).

To this is added 25% (vol.) hydroxyapatite (HAP) calculated on the polyethylene. The suspension is heated to 130°C, in which heating process a homogeneous HAP dispersion is being formed in an UHMWPE solution in xylene. The solution is poured out into a dish at room temperature and exposed to the air for two days for evaporating the xylene. The film formed is subsequently dried in an oven at 60°C and is 0.3 mm thick. Owing to preparation process applied, the UHMWPE in the film has a low entanglement density.

Ten layers of this film are stacked in a mould and compressed at 160°C and at a pressure of 25 MPa for 15 minutes to form a plate.

The modulus of elasticity and the elongation at break of the plate are determined and the results are shown in Table 1.

Examples II-VII

The process of Example I is repeated, except that respectively 30, 40, 50, 60, 70 and 85% (vol.) hydroxyapatite is added. The results are shown in Table 1.

In fig. 1 the elongation at break in % (vertical axis) as measured in Examples I-VII is plotted as a function of the filler load in % (vol.) of hydroxyapatite (horizontal axis) calculated on the amount of polyethylene.

For the purpose of comparison are included the measuring results from J. Abram, J. Bowman, J.C. Behiri, W. Bonfield from Plastics and Rubber Processing and Applications, 4, (1984), 261- 269 determined on filled polyethylene plates produced according to the process known in the art.

The results from Examples I-VII are shown using the symbols belonging to 'A', the data of Abram et al. using the symbols belonging to 'B'.

The elongation at break of the filled polyethylene plates according to the invention has been found to be substantially higher than that from Abram et al.

## Table 1

| Example | I | II | III | IV | V | VI | VII |
|---|---|---|---|---|---|---|---|
| Modulus of elasticity (MPa) | 2611 | 4092 | 5036 | 6502 | 7084 | 8300 | * |
| Elongation at break (%) | 412 | 348 | 295 | 188 | 128 | 30 | * |

*: plate is too brittle for doing the measurements.

Example VIII

A suspension in decalin of 15% (vol.) of the polyethylene from Example I is prepared and added to it is 50% (vol.) hydroxyapatite calculated on the polyethylewne. The suspension is supplied to a twin-screw extruder, in which the suspension is dissolved and subsequently extruded at 180oC through a slit measuring 200 x 1.5 mm. The extruded tape is cooled in a water bath of 20oC and dried in an oven at 70oC.

Some pieces cut from the dried tape are stacked in a mould and compressed to form a plate under a pressure of 25 MPa at a temperature of 160oC.

The modulus of elasticity of the plate is 6 GPa and the elongation at break 200%.

Example IX

The process of Example VIII is repeated, except that the filled polyethylene is extruded in the form of a thread through a round die with a diameter of 3 mm. Pieces cut off from the thread are compressed to form a plate. The modulus of elasticity of the plate is 5.5 GPa and the elongation at break 175%.

Example X

The process of Example IX is repeated, except that after cooling the extrudate is frozen with liquid nitrogen and milled in a milling device cooled with liquid nitrogen. 90% of the particles has a size smaller than 250 $\mu$ m. The solvent is evaporated from the powder in an oven at 70$^\circ$C. Owing to the preparation process applied, the UHMWPE in the powder has a low density of entanglement. From the dried solvent-free powder a plate is pressed at a compression temperature of 160$^\circ$C.

The properties of the plate are the same as those of Example X. The powder proves to be well suited for injection moulding and extrusion processes.

Comparative Example A

Polyethylene with a normal, non-low density of entanglement and with a weight-average molecular weight of 1,500,000, mixed with 50% (vol.) hydroxyapatite calculated on the polyethylene, is suspended in a liquid which is not a solvent for polyethylene. By stirring intensively for 1 hour and subsequently evaporating the suspension agent a fine and apparently homogeneous powder mixture is obtained. This powder is compression moulded under the conditions of Example IX. In the process a very brittle plate is obtained that can easily be broken by hand, in which the HAP is found to be concentrated on the powder particle boundaries of the polyethylene.

Comparative Example B

According to the process of Comparative Example A a powder mixture is made of polyethylene with a weight-average molecular weight of 600,000 and 10% (vol.) HAP calculated on the polyethylene. Attempts are made to extrude this mixture in an extruder having a screw geometry that can be varied easily. There is no screw geometry whatsoever by which a continuous extrusion process can be realized, so that it is impossible to produce a homogeneous mixture of polyethylene and hydroxyapatite, or an article thereof.

**Claims**

1. Prosthesis from polyethylene filled with an inorganic filler, characterized in that the prosthesis is formed from a composition comprising ultra-high molecular weight polyethylene with a low entanglement density and the inorganic filler.

2. Prosthesis according to claim 1, characterized in that the inorganic filler is substantially a calcium salt.

3. Prosthesis according to claim 2, characterized in that the calcium salt comprises synthetic or natural calcium phosphate, hydroxyapatite or fluorapatite.

4. Prosthesis according to any one of claims 1-3, characterized in that the weight-average molecular weight of the polyethylene is at least 500,000 kg/kmole.

5. Prosthesis according to any one of claims 1-3, characterized in that the weight-average molecular weight of the polyethylene is at least 1,000,000 kg/kmole.

6. Prosthesis according to any one of claims 1-5, characterized in that the prosthesis is built up from parts having different filler loads.

7. Process for producing a prosthesis according to any one of claims 1-6 by moulding an ultra-high molecular weight polyethylene with a low density of entanglement, filled with an inorganic filler, at a temperature of between $T_m$-25$^\circ$C and $T_m$+60$^\circ$C where $T_m$ is the melting point of the filled ultra-high molecular weight polyethylene.

8. Process according to claim 7, characterized in that the prosthesis is built up from separate parts preformed at a temperature of between $T_m$-25$^\circ$C and $T_m$ by compressing these parts at a temperature of between $T_m$ and $T_m$+60$^\circ$C.

9. Process according to claim 8, characterized in that the filler loads of the separate parts differ from each other.

10. Process according to any one of claims 7-9, characterized in that the prosthesis or the separate parts are produced from ultra-high molecular weight polyethylene powder with a low density of entanglement, filled with inorganic filler.

11. Process according to claim 10, characterized in that the particle size of the filled polyethylene powder is between 50 $\mu$ m and 500 $\mu$ m for at least 90% of the particles.

12. Composition in the form of a powder comprising ultra-high molecular weight polyethylene with a low density of entanglement, filled with an inorganic filler.

13. Composition in the form of a powder according to claim 12, characterized in that the powder is suited for the production of a prosthesis according to any one of claims 1-6.

FIG. 1

EP 0 472 237 A1

European
Patent Office

EUROPEAN SEARCH
REPORT

Application Number

EP 91 20 2075

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 373 800 (DU PONT DE NEMOURS) − − − | | A 61 L 27/00 |
| A | EP-A-0 215 507 (STAMICARBON) − − − | | C 08 L 23/06 // (C 08 L 23/06 |
| A | EP-A-0 292 074 (STAMICARBON) − − − − − | | C 08 K 3:32 ) |

| TECHNICAL FIELDS SEARCHED (Int. Cl.5) |
|---|
| A 61 L |
| C 08 L |

The present search report has been drawn up for all claims

| Place of search | Date of completion of search | Examiner |
|---|---|---|
| The Hague | 27 September 91 | PELTRE CHR. |